# EUROPEAN PATENT SPECIFICATION

(11) **EP 1 875 902 B1**
(45) Date of publication and mention of the grant of the patent: **06.11.2013**
(21) Application number: 06731704.0
(22) Date of filing: 12.04.2006
(51) Int. Cl.: A61K 31/09, A61K 8/33, A61Q 13/00, A61P 25/20, D01F 1/10, C11B 9/00, D06M 13/00, A61Q 5/02, A61Q 19/00, A61Q 19/10, D01F 2/04

(54) **SEDATIVE EFFECT-PROVIDING AGENT AND SEDATIVE FRAGRANCE COMPOSITION CONTAINING THE SAME**
MITTEL MIT SEDIERENDER WIRKUNG UND DIESES ENTHALTENDE BERUHIGENDE DUFTZUSAMMENSETZUNG
AGENT SEDATIF ET COMPOSITION SEDATIVE QUI LE CONTIENT LIBERANT UNE FRAGRANCE

(30) Priority: 13.04.2005 JP 2005115514
(43) Date of publication of application: 09.01.2008
(73) Proprietor: SHISEIDO COMPANY, LTD., Tokyo 104-8010 (JP)
(72) Inventor: MORI, Keiko SHISEIDO Research Center (Shin-Yokohama), Yokohama-shi, Kanagawa 2248558 (JP); TERAJIMA, Y., SHISEIDO Research Center (Shin-Yokohama), Yokohama, Kanagawa 2248558 (JP); YOMOGIDA, K. SHISEIDO Research Center (Shin-Yokohama), Yokohama-shi, Kanagawa 2248558 (JP); YOSHIMURA, M. SHISEIDO Research Center (Shin-Yokohama), Yokohama-shi, Kanagawa 2248558 (JP); HAZE, S. SHISEIDO Research Center (Shin-Yokohama), Yokohama, Kanagawa 2248558 (JP)
(74) Representative: Henkel, Breuer & Partner
(86) International application number: PCT/JP2006/307768
(87) International publication number: WO 2006/112334

(56) References cited:
- EP-A2- 0 988 856
- WO-A1-02/05772
- WO-A1-97/34987
- WO-A1-97/34988
- WO-A1-97/34993
- WO-A1-02/090479
- WO-A1-03/072078
- WO-A1-2005/048964
- WO-A2-98/27261
- DE-A1- 10 212 687
- DE-U1- 29 923 781
- JP-A- 2000 086 478
- JP-A- 2003 137 758
- JP-A- 2003 206 237
- JP-A- 2003 300 811
- US-A1- 2003 166 497
- US-A1- 2005 192 207
- US-B1- 6 344 218
- DATABASE BIOSIS [Online] BIOSCIENCES INFORMATION SERVICE, PHILADELPHIA, PA, US; 1 September 2002 (2002-09-01), ROGERSON FRANK S S ET AL: "1,3-dimethoxybenzene, a newly identified component of port wine" XP002597991 Database accession no. PREV200200513467 & JOURNAL OF THE SCIENCE OF FOOD AND AGRICULTURE, vol. 82, no. 11, 1 September 2002 (2002-09-01), pages 1287-1292, ISSN: 0022-5142
- DATABASE WPI Week 199228 Thomson Scientific, London, GB; AN 1992-232021 XP002597992 & JP 4 159399 A (SHISEIDO CO LTD) 2 June 1992 (1992-06-02)
- DATABASE MEDLINE [Online] US NATIONAL LIBRARY OF MEDICINE (NLM), BETHESDA, MD, US; September 1989 (1989-09), MOROKHOEV V I: "[Play olfactometry for children]" XP002597993 Database accession no. NLM2588414 & VESTNIK OTORINOLARINGOLOGII 1989 SEP-OCT LNKD- PUBMED:2588414, no. 5, September 1989 (1989-09), pages 43-45, ISSN: 0042-4668
- DATABASE MEDLINE [Online] US NATIONAL LIBRARY OF MEDICINE (NLM), BETHESDA, MD, US; September 2000 (2000-09), SEITZ L M ET AL: "Volatile methoxybenzene compounds in grains with off-odors." XP002597994 Database accession no. NLM10995350 & JOURNAL OF AGRICULTURAL AND FOOD CHEMISTRY SEP 2000 LNKD- PUBMED:10995350, vol. 48, no. 9, September 2000 (2000-09), pages 4279-4289, ISSN: 0021-8561
- "m-dimethoxybenzene" FOOD AND CHEMICAL TOXICOLOGY, PERGAMON, GB, vol. 38, 1 January 2000 (2000-01-01), pages S59-S62, XP025950015 ISSN: 0278-6915 [retrieved on 2000-01-01]
- DATABASE WPI Week 198215 Thomson Scientific, London, GB; AN 1982-29828E XP002597995 & JP 57 040402 A (IDEMITSU KOSAN CO LTD) 6 March 1982 (1982-03-06)
- DATABASE BIOSIS [Online] BIOSCIENCES INFORMATION SERVICE, PHILADELPHIA, PA, US; 1991, GERLACH G ET AL: "COMPOSITION OF ORCHID SCENTS ATTRACTING EUGLOSSINE BEES" XP002597996 Database accession no. PREV199293069512 & BOTANICA ACTA, vol. 104, no. 5, 1991, pages 379-391, ISSN: 0932-8629
- KIER L.B. ET AL.: 'Molecular Connectivity. 4. Relationships to Biological Activities' JOURNAL OF MEDICINAL CHEMISTRY vol. 18, no. 12, 1975, pages 1272 - 1274, XP003003246

## Description

### . TECHNICAL FIELD

The present invention relates to methods for imparting a sedative effect and the non-therapeutic use of a sedative effect-imparting agent, particularly to improvement in fragrance-releasing property of a perfume component that provides a sedative effect.

### BACKGROUND ART

It has been traditionally confirmed that an essential oil such as lavender or chamomile oil provides sedative effect in the field of aromatherapy. Such essential oils can provide sedative effect by inhalation thereof. Thus, the inhalation administration has an advantage that it does not demand additional stress compared, for example, with oral administration or injection administration. However, the essential oils are mixture of various kinds of perfume components and contain several kinds of non-sedative components as well, therefore a large amount of essential oil is required for favorable result. This leads to significant fluctuation in the preference by individuals to a particular fragrance, causing a problem that a particular fragrance does not provide favorable action to some people, and yet provide them with adverse effects. On the other hand, it was reported recently that a single perfume component, for example dimethoxymethylbenzene (see, for example, Patent Document 1) or a trialkoxybenzene such as trimethoxybenzene (see, for example, Patent Document 2), provided a sedative effect.

Patent Document 1: Japanese Unexamined Patent Publication No. 6-172781
Patent Document 2: Japanese Unexamined Patent Publication No. 2000-86478 JP 4 159 399 A discloses a perfume composition having a lotus-like fragrance by using 1,4-dimethoxybenzene as the constituent and containing at least 10% by weight, preferably at least 20% by weight of 1,4-dimethoxybenzene. The composition can be used as a perfume, an eau de cologne or an interior aromatic.
Medline abstract NLM2588414 (Morokhoev, VESTNIK OTORINOLARINGOLOGII (1989), 5, 43-45) suggests a 50% aqueous solution of dimethoxybenzene as an olfactory stimulus.
DE 102 12 687 A1 provides a perfume composition containing 1,4-dimethoxybenzene and another fragrance, whereby the content of both fragrances taken together is at least 70% by weight.
WO 02/090479 A1 is directed to a perfume delivery composition comprising perfumed particles comprising a solid, water insoluble, porous mineral carrier and a perfume composition incorporated therein. The perfume composition can contain veratrol (1,2-dimethoxybenzene). Also disclosed is a solid fabric conditioning composition comprising said perfumed particles.
JP 57 040 402 A discloses an insecticide obtained by blending naphthalene and a sublimate polar compound with an insecticidal substance, whereby the sublimate polar compound is employed in an amount of 0.2-5 parts by weight based on 100 parts by weight of naphthalene and can be dimethoxybenzene.
JP 5230495 A provides a fragrant cosmetic containing an essential oil for sedation, which is made by removing high-boiling components from jasmine oil.
JP 11343497 A discloses a cosmetic for relieving stress by including a perfume component having a tranquillizing effect and causing a quiet sleep such as one or more of perfume components of rosemary, lavender, chamomile blue, bitter orange and rhizome of Cnidium officinale.

### SUMMARY OF THE INVENTION

### Problems to be Solved by the Invention

However, sedative perfume component described above as dimethoxymethylbenzene or trialkoxybenzene has not enough volatility, i.e. fragrance-releasing property, causing a problem that it was difficult to obtain the sedative effect immediately in practical use. On the other hand, increase in blending amount of the component in a perfume composition for improvement of sedative effect, unfavorably resulted in change in the fragrance tone of the entire perfume composition. Also, when the dose of entire perfume composition is increased, it causes excessive odor intensity. For the reasons above, there was a need for development of a new sedative effect-imparting agent with good fragrance-releasing property, to solve these problems.

The present invention was carried out to solve such problems in the prior art. An object of the present invention is to provide a sedative effect-imparting agent that provides excellent sedative effect with good fragrance-releasing property, and is possible to provide the excellent sedative effect immediately.

### Means to Solve the Problems

The present inventors have diligently researched to solve the problems as above. As a result, the present inventors have found that it is possible to provide more favorable sedative effect by volatilization and inhalation of dimethoxybenzene, and dimethoxybenzene provides better fragrance-releasing property than the conventional sedative perfume components, such as dimethoxymethylbenzene and trialkoxybenzenes, and it is possible to provide more favorable sedative effect immediately, thus leading to completion of the present invention.

Accordingly, one aspect of the present invention is a non-therapeutic use of dimethoxybenzene for imparting a sedative effect. Preferably, the sedative effect-imparting agent, i.e. dimethoxybenzene, is used when contained in a sedative perfume composition in an amount of 0.01 to 50 mass %.

A further aspect of the present invention is a method for imparting a sedative effect to a cosmetic or an article of clothing by incorporating the sedative effect-imparting agent, i.e. dimethoxybenzene, into a cosmetic or an article of clothing, respectively.

### EFFECT OF THE INVENTION

The sedative effect-imparting agent used according to the present invention provides excellent sedative effect immediately, since it consists of dimethoxybenzene that provides excellent sedative effect with good fragrance-releasing property.

### BRIEF DESCRIPTION OF THE DRAWINGS

Figure 1 is a graph showing the results obtained in CNV measurement of o-dimethoxybenzene (o-DMB), m-dimethoxybenzene (m-DMB), and p-dimethoxybenzene (p-DMB).
Figure 2 is a graph showing the results obtained in headspace GCMS analysis of a sample solution containing an equal amount of 5 kinds of perfume components: o-dimethoxybenzene (o-DMB), m-dimethoxybenzene (m-DMB), p-dimethoxybenzene (p-DMB), 1,3-dimethoxy-5-methylbenzene (DMMB), and 1,3,5-trimethoxybenzene (TMB).

### DESCRIPTION OF THE PREFERRED EMBODIMENTS

Hereinafter, the present invention will be described in more detail.
The sedative effect-imparting agent used according to the present invention consists of dimethoxybenzene represented by the following Formula (1).

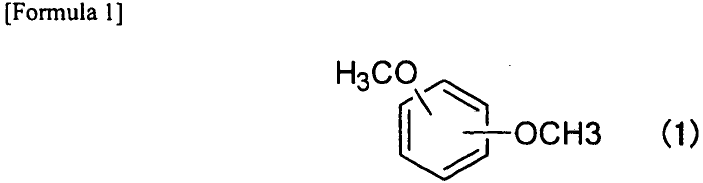

Regarding dimethoxybenzene used in the present invention, two methoxy groups may be substituted at either o-, m-, or p-positions on the benzene ring, preferably at o- or m-positions. The fragrances of respective dimethoxybenzenes are different from each other. o-dimethoxybenzene has sweet cream-like fragrance, or sweet vanillin-like fragrance when diluted; m-dimethoxybenzene has strong hazelnut-like fragrance; and p-dimethoxybenzene has sweet coumarin-, nut-, or withered grass-like fragrance and is also known as a characteristic component presented in a lotus flower "Maihiren" (Synthetic Fragrances, Chemistry and Product Information, Edited by Motoichi Indo, The Chemical Daily Co., Ltd.).

Further, the sedative perfume composition used according to the present invention is a combination of the sedative effect-imparting agent used according to the present invention, i.e., dimethoxybenzene, with any other perfume components. The sedative perfume composition used according to the present invention contains 0.01 to 50 mass % of dimethoxybenzene, as an active ingredient, with respect to the total amount of the perfume composition. The content of dimethoxybenzene is particularly preferably 0.1 to 10 mass %. Unfavorably, a dimethoxymethylbenzene content of 0.01 mass % or less may result in insufficient sedative effect, while a dimethoxymethylbenzene content of 50 mass % or more does not lead to significant improvement in its sedative effect, but only to unbalance of dimethoxymethylbenzene with other perfume components. The sedative perfume composition used according to the present invention can be used as perfume, cologne, as itself or its dilution with a suitable solvent.

The sedative effect-imparting agent used according to the present invention may be used in combination with other blending components as necessary, in cosmetic preparations such as shampoo/rinse, skincare cosmetics, body shampoo, body rinse, body powder, air freshener, deodorant, and bath preparations. Further, the sedative effect-imparting agent used according to the present invention may be used in adding fragrance to an article of clothing.

Hereinafter, favorable embodiments of the present invention will be described in more detail.
First, a test method for determining the sedative effect in the present invention will be described.
Measured was fluctuation in the negative potential of the event-related potential, a type of brain wave, which is also called contingent negative variation (hereinafter, referred to as CNV). The fluctuation intensity of the earlier component of the CNV brain wave is reported to show a positive correlation with the level of consciousness such as attention or expectation, and thus, the CNV brain wave can be used for quantitative evaluation of the effect of a fragrance on the consciousness level (sedated/stimulated) (Torii S. et al., Contingent negative variation (CNV) and the psychological effects of odour. In Perfumery: The Psychology and Biology of Fragrance, Edited by Toller S.V. and Dodd G.H., pp.107-120, Chapman and Hall, London (1998)).

In the present invention, electrodes were adhered to the forehead (Cz) and the left ear (A1) of a subject, and the voltage between the electrodes was measured with an electroencephalograph. The subject was made to listen to a click sound via headphone and push a switch in hand in response to a light-emitting diode flashing few seconds after the click sound, and the CNV brain wave was determined during the operation. In the test with fragrance, a perfume composition was allowed to penetrate into a square cotton pad of approximately 0.5×0.5 cm in size; the cotton pad was placed below the nose; and the subject was allowed to inhale the fragrance together with normal breathing during the series of operations above. In the test without fragrance, a square cotton pad without the perfume composition of approximately 0.5×0.5 cm in size was placed below the nose; and the subject was made to perform the same operation. The CNV brain waves were measured 30 times with and without fragrance respectively, and the fragrance effect was evaluated by comparing the integrated values of the earlier component of the CNV brain wave 400 to 1,000 ms after the click.

### Sedative effect

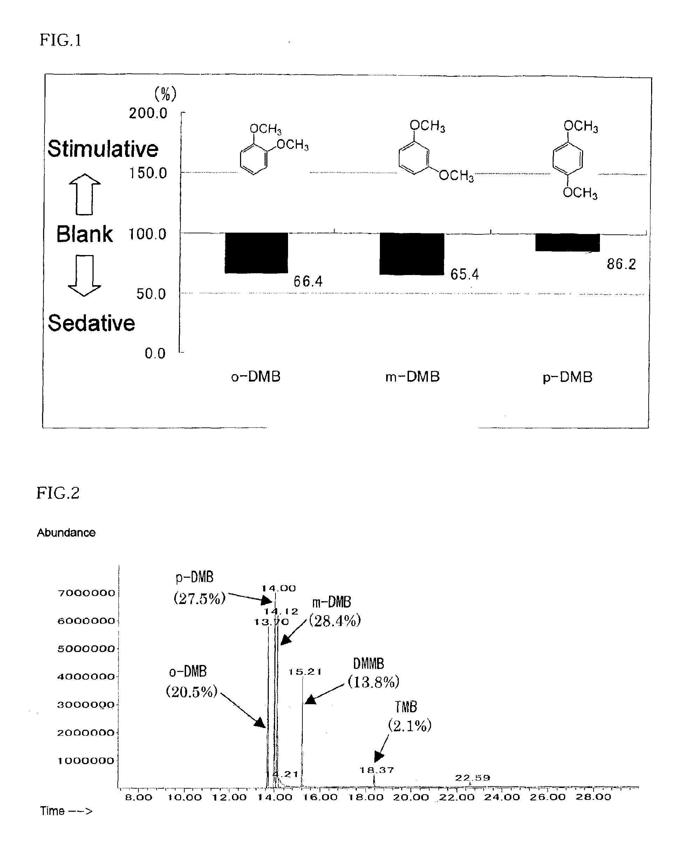

The inventors first examined the sedative effect of the dimethoxybenzenes by the test method described above.
o-dimethoxybenzene (o-DMB), m-dimethoxybenzene (m-DMB), and p-dimethoxybenzene (p-DMB) were dissolved respectively in ethanol, to give 1 mass % solutions, and the effect thereof on CNV was measured according to the test method as above. The test was performed by three to six healthy adult females as panel. Results are summarized in Figure 1.
As obvious from Figure 1, any one of o-, m-, and p-dimethoxybenzenes shows a favorable effect that leads the subject to sedative direction. It is also obvious that o- and m-dimethoxybenzenes exhibit particularly favorable sedative effect.

### Fragrance-releasing property

The inventors then evaluated the fragrance-releasing property of the various dimethoxybenzenes.
For comparison of fragrance-releasing property of various perfume components, triethyl citrate solution with 5 kinds of perfume components, i.e., o-dimethoxybenzene (o-DMB), m-dimethoxybenzene (m-DMB), p-dimethoxybenzene (p-DMB), 1,3-dimethoxy-5-methylbenzene (DMMB), and 1,3,5-trimethoxybenzene (TMB), were prepared (2 mass % mixture, respectively). And the perfume components vaporized therefrom were analyzed by a headspace GCMS. Results were summarized in Figure 2.

Analytical conditions for the headspace GCMS are as follows:
GC-MS apparatus: manufactured by Agilent Technologies
GC analysis condition
Carrier gas: helium
Flow rate: 1.2 ml/min
Column: HP-INNOWAX (PEG system) [0.25 mmϕ (ID) × 60 m (L)]
Column temperature: 60 (0)-> 230 (13) (heating rate: 10°C/min)
* ( ): retention time (minute) at each temperature
Splitless injection
·MS analysis condition (quadrupole mass spectrometer)
Ionization method: EI (70 eV), ·Detection: TIC
·Headspace sampling (perfume component solution: 0.5 g)
The fiber assembly for solid-phase micro-extraction: CAR/PDMS type (SPME fiber, manufactured by Supelco)
Sampling in the headspace vial container for 10 minutes

As evidenced by Figure 2, although each perfume component was dissolved in the solution in the same amount (2%), the amount of the perfume component vaporized into the headspace varied significantly according to the kind of the perfume component. Three kinds of dimethoxybenzenes (DMBs) showed higher volatility than known sedative perfume components, 1,3-dimethoxy-5-methylbenzene (DMMB) and 1,3,5-trimethoxybenzene (TMB). Thus, this indicates that the DMBs have better fragrance-releasing property and can be vaporized and inhaled by human in a shorter period of time. Accordingly, use of the dimethoxybenzene as a sedative effect-imparting agent is giving more favorable sedative effect immediately, compared with conventional DMMB and TMB.

### [Example 1]

Hereinafter, favorable examples of the present invention will be described in detail.

**[Table 1]**

| Example 1-1: Floral sedative perfume composition (containing o-DMB) | mass % |
|---|---|
| Citronellol | 10 |
| Citronellyl acetate | 3 |
| Geraniol | 15 |
| Geranyl acetate | 5 |
| cis-3-Hexenol | 0.2 |
| Nerol | 3 |
| Phenylethyl alcohol | 40 |
| Phenylethyl acetate | 10 |
| Eugenol | 1 |
| Dipropylene glycol | 7.8 |
| o-dimethoxybenzene | 5 |
| Total | 100 |

**[Table 2]**

| Example 1-2: Floral sedative perfume composition (containing p-DMB) | mass % |
|---|---|
| cis-3-Hexenol | 0.2 |
| Decanal | 0.1 |
| 1,8-Cineole | 1 |
| Methyl benzoate | 5 |
| Methyl salicylate | 0.4 |
| Linalool | 15 |
| Methyl dihydrojasmonate | 30 |
| Citronellol | 5 |
| Geraniol | 5 |
| Phenylethyl alcohol | 20 |
| Alpha-terpineol | 4 |
| cis-Jasmone | 1 |
| Helional (manufactured by IFF) | 1 |
| Dipropylene glycol | 7.3 |
| p-dimethoxybenzene | 5 |
| Total | 100 |

**[Table 3]**

| Example 1-3: Floral sedative perfume composition (containing m-DMB) | mass % |
|---|---|
| Peach base | 1 |
| Apple base | 3 |
| Jasmine base | 15 |
| Rose base | 5 |
| Lilial (manufactured by Givaudan) | 10 |
| Methyl dihydrojasmonate | 25 |
| Methyl ionone | 10 |
| Cyclopentadecanolide | 5 |
| Musk T | 5 |
| Dipropylene glycol | 16 |
| m-dimethoxybenzene | 5 |
| Total | 100 |

### [Example 2]

**[Table 4]**

| Example 2-1: Fragrance | mass % |
|---|---|
| Alcohol | 75 |
| Purified water | Balance |
| Dipropylene glycol | 5 |
| Sedative perfume composition of Example 1-1 | 10 |
| Antioxidant | q.s. |
| Grapefruit oil | 5 |
| Colorant | q.s. |
| Total | 100 |

**[Table 5]**

| Example 2-2: Skin lotion | mass % |
|---|---|
| Glycerin | 2 |
| Dipropylene glycol | 2 |
| PEG-60 hydrogenated castor oil | 0.3 |
| Xylitol | 3 |
| Ascorbic acid | 0.005 |
| Trisodium EDTA | 0.1 |
| Dye | q.s. |
| Sedative perfume composition of Example 1-2 | q.s. |
| Purified water | Balance |
| Total | 100 |

**[Table 6]**

| Example 2-3: Skin lotion | mass % |
|---|---|
| Alcohol | 30 |
| Butylene glycol | 4 |
| Glycerin | 2 |
| PPG-13 Decyltetrades 24 | 0.3 |
| Octylmethoxycinnamate | 0.1 |
| Menthol | 0.2 |
| Tranexamic acid | 1.0 |
| Trisodium EDTA | 0.1 |
| Dye | q.s. |
| Sedative perfume composition of Example 1-3 | q.s. |
| Purified water | Balance |
| Total | 100 |

**[Table 7]**

| Example 2-4: Emulsion | mass % |
|---|---|
| Ethyl alcohol | 10 |
| Glycerin | 3 |
| Butylene glycol | 2 |
| Polyethylene glycol | 3 |
| Carboxyvinyl polymer | 0.1 |
| Acrylic acid/alkyl acrylate copolymer | 0.1 |
| Caustic potash | 0.1 |
| Cyclomethicone | 4 |
| Squalane | 2 |
| Spherical polyethylene | 2 |
| Menthol | 0.5 |
| Active ingredient | q.s. |
| Paraben | q.s. |
| Trisodium EDTA | 0.1 |
| Pigment | q.s. |
| Sedative perfume composition of Example 1-1 | q.s. |
| Purified water | Balance |
| Total | 100 |

**[Table 8]**

| Example 2-5: Emulsion | mass % |
|---|---|
| Butylene glycol | 4 |
| Propylene glycol | 4 |
| Carboxyvinyl polymer | 0.2 |
| Caustic potash | 0.2 |
| Behenic acid | 0.5 |
| Stearic acid | 0.5 |
| Isostearic acid | 0.5 |
| Glyceryl stearate | 1 |
| Glyceryl isostearate | 1 |
| Behenyl alcohol | 0.5 |
| Batyl alcohol | 0.5 |
| Squalane | 5 |
| Trioctanoin | 3 |
| Phenyl trimethicone | 2 |
| Beech bud extract | 0.5 |
| Phenoxyethanol | q.s. |
| Trisodium EDTA | 0.1 |
| Pigment | q.s. |
| Sedative perfume composition of Example 1-2 | q.s. |
| Purified water | Balance |
| Total | 100 |

**[Table 9]**

| Example 2-6: Emulsion | mass % |
|---|---|
| Glycerin | 3 |
| Xylitol | 2 |
| Carboxyvinyl polymer | 0.1 |
| Caustic potash | 0.1 |
| Glyceryl isostearate | 1 |
| Glyceryl stearate | 0.5 |
| Behenyl alcohol | 1 |
| Batyl alcohol | 1 |
| Hydrogenated palm oil | 2 |
| Vaseline | 1 |
| Squalane | 5 |
| Erythrityl octanoate | 3 |
| Cyclomethicone | 1 |
| Magnesium ascorbyl phosphate | 0.5 |
| Paraben | q. s. |
| Trisodium EDTA | 0.1 |
| Sedative perfume composition of Example 1-3 | Balance |
| Purified water | Balance |
| Total | 100 |

**[Table 10]**

| Example 2-7: Cream | mass % |
|---|---|
| Glycerin | 10 |
| Butylene glycol | 5 |
| Carboxyvinyl polymer | 0.1 |
| Caustic potash | 0.2 |
| Stearic acid | 2 |
| Glyceryl stearate | 2 |
| Glyceryl isostearate | 2 |
| Vaseline | 5 |
| Stearyl alcohol | 2 |
| Behenyl alcohol | 2 |
| Hydrogenated palm oil | 2 |
| Squalane | 10 |
| α-Glucosylhesperidin | 0.1 |
| Paraben | q.s. |
| Trisodium EDTA | 0.1 |
| Pigment | q.s. |
| Sedative perfume composition of Example 1-3 | q.s. |
| Purified water | Balance |
| Total | 100 |

**[Table 11]**

| Example 2-8: Cream | mass % |
|---|---|
| Glycerin | 3 |
| Dipropylene glycol | 7 |
| Polyethylene glycol | 3 |
| Glyceryl stearate | 3 |
| Glyceryl isostearate | 2 |
| Stearyl alcohol | 2 |
| Behenyl alcohol | 2 |
| Liquid paraffin | 7 |
| Cyclomethicone | 3 |
| Dimethicone | 1 |
| Octylmethoxycinnamate | 0.1 |
| Vitamin A acetate | 0.5 |
| Phenoxyethanol | q.s. |
| Trisodium EDTA | 0.1 |
| Pigment | q.s. |
| Sedative perfume composition of Example 1-2 | q.s. |
| Purified water | Balance |
| Total | 100 |

**[Table 12]**

| Example 2-9: Gel | mass % |
|---|---|
| Ethyl alcohol | 10 |
| Glycerin | 5 |
| Butylene glycol | 5 |
| Carboxyvinyl polymer | 0.5 |
| AMP | 0.3 |
| PEG-60 hydrogenated castor oil | 0.3 |
| Menthol | 0.02 |
| Oil-soluble licorice extract (root) | 0.2 |
| Paraben | q.s. |
| Trisodium EDTA | 0.1 |
| Sedative perfume composition of Example 1-1 | q.s. |
| Purified water | Balance |
| Total | 100 |

**[Table 13]**

| Example 2-10: Aerosol | mass % |
|---|---|
| Glycerin | 2 |
| Dipropylene glycol | 2 |
| PEG-60 hydrogenated castor oil | 0.3 |
| Equisetum giganteum (radical leaf) | 0.5 |
| Paraben | q.s. |
| Trisodium EDTA | 0.1 |
| Dye | q.s. |
| Sedative perfume composition of Example 1-2 | q.s. |
| Purified water | Balance |
| Nitrogen gas | 0.8 |
| Total | 100 |

**[Table 14]**

| Example 2-11: Aerosol | mass % |
|---|---|
| Alcohol | 15 |
| Butylene glycol | 2 |
| Glycerin | 1 |
| PPG-13 Decyltetrades 24 | 0.1 |
| Potassium 4-methoxysalicylate | 0.5 |
| Trisodium EDTA | 0.1 |
| Dye | q.s. |
| Sedative perfume composition of Example 1-1 | q.s. |
| Purified water | Balance |
| LPG | 40 |
| Total | 100 |

**[Table 15]**

| Example 2-12: Bath articles | mass % |
|---|---|
| Sodium sulfate | 45 |
| Sodium hydrogencarbonate | 45 |
| Hyssop oil | 10 |
| Sedative perfume composition of Example 1-3 | q.s. |
| Total | 100 |

**[Table 16]**

| Example 2-13: Room fragrance | mass % |
|---|---|
| Alcohol | 80 |
| Purified water | Balance |
| Antioxidant | 5 |
| Sedative perfume composition of Example 1-2 | q.s. |
| Clary sage oil | 5 |
| Total | 100 |

**[Table 17]**

| Example 2-14: Incense | mass % |
|---|---|
| Tabu powder | 75 |
| Sodium benzoate | 15 |
| Sedative perfume composition of Example 1-3 | q.s. |
| Purified water | Balance |
| Total | 100 |

**[Table 18]**

| Example 2-15: Foam pack | mass % |
|---|---|
| Caffeine | 1 |
| Sodium metaphosphate | 0.02 |
| Trehalose | 2 |
| Glycerin | 7 |
| Methylparaben | 0.1 |
| Potassium hydroxide | 0.15 |
| Stearic acid | 0.5 |
| Myristic acid | 1 |
| Batyl alcohol | 1.5 |
| Polyoxyethylene (60) hydrogenated castor oil | 3 |
| Fennel oil | 0.3 |
| Liquefied petroleum gas | 6 |
| Dimethylether | 3 |
| Sedative perfume composition of Example 1-1 | q.s. |
| Purified water | Balance |
| Total | 100 |

**[Table 19]**

| Example 2-16: Shampoo | mass % |
|---|---|
| Lauryl polyoxyethylene (3) sulfate ester sodium salt | 10 |
| Laurylsulfate ester sodium salt | 5 |
| Coconut oil fatty acid diethanolamide | 4 |
| Glycerin | 1 |
| Sedative perfume composition of Example 1-3 | q.s. |
| Colorant | q.s. |
| Paraben | q.s. |
| Trisodium EDTA | 0.1 |
| Citric acid | 0.05 |
| Sodium citrate | 0.05 |
| Purified water | Balance |
| Total | 100 |

**[Table 20]**

| Example 2-17: Shampoo | mass % |
|---|---|
| Lauryl polyoxyethylene (3) sulfate ester triethanolamine salt | 5 |
| Lauryl polyoxyethylene (3) sulfate ester sodium salt | 5 |
| Laurylsulfate ester sodium salt | 5 |
| Lauroyl monoethanolamide | 1 |
| Lauryldimethylaminoacetic acid betaine | 5 |
| Cationic cellulose | 7 |
| Ethylene glycol distearate ester | 2 |
| Protein derivative | 0.5 |
| Sedative perfume composition of Example 1-2 | q.s. |
| Trisodium EDTA | 0.1 |
| Citric acid | 0.05 |
| Sodium citrate | 0.05 |
| Purified water | Balance |
| Total | 100 |

**[Table 21]**

| Example 2-18: Rinse | mass % |
|---|---|
| Silicone oil | 3 |
| Liquid paraffin | 1 |
| Cetyl alcohol | 1.5 |
| Stearyl alcohol | 1 |
| Stearyltrimethylammonium chloride | 0.7 |
| Glycerin | 3 |
| Sedative perfume composition of Example 1-3 | q.s. |
| Colorant | q.s. |
| Phenoxyethanol | q.s. |
| Purified water | Balance |
| Total | 100 |

### Example 2-19: Fragrant fiber

To a cupro ammonium cellulose solution (cellulose concentration: 10 mass %, ammonium concentration: 7 mass %, copper concentration: 3.6 mass %), microcapsules encapsulating a sedative perfume composition obtained in Example 1-1 (particle size: not greater than 50 µm, ratio of essential oil in microcapsule: 50 mass %) were added in an amount in the range of 0.1 to 20 mass % with respect to cellulose, and after mixing, the resulting mixture was processed by a common wet spinning method and then in purification and drying steps, to give a fragrant fiber.

## Claims

1. A method for imparting a sedative effect to a cosmetic comprising a step of incorporating dimethoxybenzene into a cosmetic.

2. The method according to claim 1, wherein the dimethoxybenzene is contained in a sedative perfume composition in an amount of 0.01 to 50 mass % with respect to the total amount of the perfume composition.

3. A method for imparting a sedative effect to an article of clothing comprising a step of incorporating dimethoxybenzene into an article of clothing.

4. A non-therapeutic use of dimethoxybenzene for imparting a sedative effect.

5. The use according to claim 4, wherein the dimethoxybenzene is contained in a sedative perfume composition in an amount of 0.01 to 50 mass %.

## Patentansprüche

1. Verfahren zum Verleihen einer sedierenden Wirkung an ein Kosmetikum, wobei das Verfahren eine Stufe des Einarbeitens von Dimethoxybenzol in ein Kosmetikum umfasst.

2. Verfahren nach Anspruch 1, wobei das Dimethoxybenzol in einer sedierenden Duftstoffzusammensetzung in einer Menge von 0,01 bis 50 Masse-%, bezogen auf die Gesamtmenge der Duftstoffzusammensetzung, enthalten ist.

3. Verfahren zum Verleihen einer sedierenden Wirkung an einen Bekleidungsgegenstand, wobei das Verfahren eine Stufe des Einarbeitens von Dimethoxybenzol in einen Bekleidungsgegenstand umfasst.

4. Nichttherapeutische Verwendung von Dimethoxybenzol zum Verleihen einer sedierenden Wirkung.

5. Verwendung nach Anspruch 4, wobei das Dimethoxybenzol in einer sedierenden Duftstoffzusammensetzung in einer Menge von 0,01 bis 50 Masse-% enthalten ist.

## Revendications

1. Procédé pour conférer un effet sédatif à un cosmétique comprenant une étape d'incorporation de diméthoxybenzène dans un cosmétique.

2. Procédé selon la revendication 1, dans lequel le diméthoxybenzène est contenu dans une composition de parfum sédative en une quantité de 0,01 à 50 % en masse par rapport à la quantité totale de la composition de parfum.

3. Procédé pour conférer un effet sédatif à un article de vêtement, comprenant une étape d'incorporation de diméthoxybenzène dans un article de vêtement.

4. Utilisation non thérapeutique de diméthoxybenzène pour conférer un effet sédatif.

5. Utilisation selon la revendication 4, dans laquelle le diméthoxybenzène est contenu dans une composition de parfum sédative en une quantité de 0,01 à 50 % en masse.
